# EUROPEAN PATENT SPECIFICATION

(11) **EP 2 398 458 B1**
(45) Date of publication and mention of the grant of the patent: **17.07.2013**
(21) Application number: 10711743.4
(22) Date of filing: 19.02.2010
(51) Int. Cl.: A61K 9/00, A61K 31/716, A61K 33/38, A61K 45/06

(54) **PHARMACEUTICAL COMPOSITION CONTAINING MICROCOLLOIDAL SILVER AND CARBOXYMETHYL BETA GLUCAN FOR DISINFECTION OF THE NASAL MUCOSAE**
PHARMAZEUTISCHE ZUSAMMENSETZUNG MIT MIKROKOLLOIDALEM SILBER UND CARBOXYMETHYL-BETA-GLUCAN ZUR DESINFEKTION DER NASENSCHLEIMHAUT
COMPOSITION PHARMACEUTIQUE À BASE D'ARGENT MICRO-COLLOÏDAL ET DE BÊTA-GLYCANE DE CARBOXY-MÉTHYLE DESTINÉ À LA DÉSINFECTION DES MUQUEUSES NASALES

(30) Priority: 20.02.2009 IT RM20090077
(43) Date of publication of application: 28.12.2011
(73) Proprietor: D.M.G. Italia Srl, 00040 Pomezia (RM) (IT)
(72) Inventor: MERCURI1 Luigi, 1-00040 Pomezia (RM) (IT)
(74) Representative: Sarpi, Maurizio
(86) International application number: PCT/IT2010/000064
(87) International publication number: WO 2010/095158

(56) References cited:
- US-A1- 2006 122 082
- US-A1- 2008 020 060
- US-B1- 6 454 754
- KUBALA L ET AL: "The effect of (1->3)-beta-d-glucans, carboxymethylglucan and schizophyllan on human leukocytes in vitro" CARBOHYDRATE RESEARCH, ELSEVIER SCIENTIFIC PUBLISHING COMPANY. AMSTERDAM, NL, vol. 338, no. 24, 21 November 2003 (2003-11-21), pages 2835-2840, XP004479501 ISSN: 0008-6215
- CHEN ET AL: "Nanosilver: A nanoproduct in medical application" TOXICOLOGY LETTERS, ELSEVIER BIOMEDICAL PRESS, AMSTERDAM, NL, vol. 176, no. 1, 16 October 2007 (2007-10-16), pages 1-12, XP022390861 ISSN: 0378-4274

## Description

The present invention relates to a pharmaceutical composition that contains an appropriate amount of microcolloidal silver and of carboxymethyl beta glucan for use in the disinfection of the nasal mucosae, according to claim 1.

The therapeutic properties of silver were already known at the times of Ancient Greece: it was in fact known that in families in which silver cutlery was used for eating, infections were less frequent.

Silver presents important antimicrobial properties since the presence of one part in 100 million of elementary silver in solution is sufficient to obtain an effective antimicrobial action. It is well known that ions or free radicals of silver are an active antimicrobial agent.

To render silver soluble in water, it is necessary to use a colloidal form (a suspension in water of extremely fine silver particles), which was obtained for the first time in 1920, by means of a process of hydrolysis in which two electrodes of pure silver are used, immersed in demineralized or distilled water, to which a difference of potential is applied.

Before the advent of antibiotics in 1938, colloidal silver thus obtained (which more appropriately should be called ionic-colloidal silver), was amply prescribed for a wide variety of diseases and infections: in fact, it had been proved that it was effective against more than 650 different pathogenic agents.

Colloidal silver enables in fact an improvement in the function of the immune system since it acts as catalyst, deactivating those specific enzymes necessary for the metabolism of many bacterial, fungine, and viral species.

With colloidal silver, the ideal concentration is indicated between 5 and 20 ppm, since a higher concentration could cause the particles, even though of the optimal size of 0.05 µm, in suspension to reunite, hence leading to a lower capacity of cellular absorption by the organism.

Interest in silver underwent an arrest with the advent of antibiotics, but starting from the seventies its use above all as antiseptic in the treatment of burns has once again been re-evaluated.

Said success derives from the fact that is a safe antiseptic; in fact, when used in colloidal form, silver is absorbed very slowly by the tissues so as not to cause toxic effects. It can hence be assumed by oral route and come to form part of those preparations that can be assumed on a daily basis both by adults and by children without danger of onset of any type of intolerance even though the use is prolonged in time.

Colloidal silver is principally used as antiseptic in dermatology for the treatment of certain types of lesions. Equally known is its application as potabilizing agent of water and as preservative in the cosmetics industry, but, on account of the high cost of production, its use is very limited.
In the otorhinolaryngological field the well recognized anti-microbial activity of silver has been widely exploited for producing several pharmaceutical compositions.
The US patent application US 2008/0020060 discloses pharmaceutical compositions comprising a colloidal suspension of nanoparticles of elemental silver to be administered in the form of spray into the nasal mucosae of a patient for controlling and treating rhinitis.
The US patent 6, 454, 754 describes a means and protocol for treating the infection of the patient respiratory tract. The device object of this invention is a nebulizer or spray means for producing a mist of appropriately sized droplets of a fluid consisting of pure silver colloid. According to the inventors such administration of ionic silver colloid makes possible more effectively treating an infection with 10,000 time less antimicrobial agent than the usually prescribed techniques.
The use of a therapeutic silver-based composition applied in medical application for preventing, treating, or reducing the spread or transmission rate of bacteria, infections, and the like is the subject-matter of the US patent application US 2006/122082 to produce a liquid foam or spray and/or gel formulations.

At this point it is necessary to make a distinction between metallic silver and silver in the form of salts.

The latter have a therapeutic action but are characterized by a high toxicity; they are in fact readily absorbed by the tissues and can hence be irritant. They may moreover lead to phenomena of accumulation, with the appearance of a bluish colouring of the skin, a pathological condition known as "*argyrosis*":

There is, for example, a rather widespread presence in the pharmaceutical field preparations with a base of salts of silver, above all silver protein and silver vitelline, used as antiseptics of the nasal cavity (Argotone, Protargol). However, on account of their toxicity and numerous side effects, their use is contraindicated in children of less than twelve years of age, is not recommended for diabetics and elderly persons, whereas for adults its use cannot be protracted for more than a week.

As regards, instead, metallic silver, precisely because it is a metal, the only possibility of use in the therapeutic field, without resorting to the process of hydrolysis, which is difficult to apply at an industrial level, envisages its micronization in particles that can be suspended in a liquid. Also this process, however, is very laborious and costly, and this has been one of the causes that has limited its production.

In fact the process of micronization must be conducted so as to produce particles that are homogeneous as regards size and must moreover be pure and stable in solution; i.e., they must not have the tendency to coagulation and hence sedimentation.

There has been developed for this purpose a stable preparation of colloidal micronized silver at concentrations not higher than 50 ppm, designed to obtain the biological activity thereof, without any side effects. A particular process of production of colloidal micronized silver starting from pure metallic silver has been developed through the use of apparatuses that enable control of particulate dimensions so as to obtain a homogeneous and stable product.

It has moreover been discovered that by mixing colloidal silver and carboxymethyl beta glucan, already known for its action of stimulus on the immune system, there is obtained an intensification of the reinforcing effect on the immune system, which can be advantageously used in the otorhinolaryngological field in the disinfection of nasal mucosae above all in children.

Carboxymethyl beta glucan is a sugar used principally as immuno-equilibrating both in topical soluble applications and in preparations for oral use. Kubala et al. in their study (Carbohydrate Research. 2003. 338: 2835-2840) have determined the immunomodulatory activity of the carboxymethylglucan (CMG) on human blood leukocytes *in vitro*. CMG has been demonstrated being able to activate blood phagocytes and lymphocytes as indicated by increased whole blood production of reactive oxygen species, by increased production of pro-inflammatory cytokines, IL-6, IL-8, and TNF-a, by increased surface expression of CD69 on lymphocytes, and by altered expression of CD11b and CD62L on polymorphonuclear leukocytes and monocytes.

Disclosed is a pharmaceutical composition constituted by a stable aqueous solution containing colloidal micronized silver and carboxymethyl beta glucan for the disinfection of the nasal mucosae, where the dispersed particles of micronized silver have a diameter of between 85 nm and 150 nm.

The colloidal micronized silver according to the present invention is preferably used in the composition in question in an amount of between approximately 10 ppm to 100 ppm and in particular in an amount of approximately 50 ppm.

Furthermore, the carboxymethyl beta glucan of the present composition is preferably present in an amount of between approximately 0.1 g and 5 g for a total of 1000 g of composition and in particular in a quantity of approximately 1 g.

Also present is at least one appropriate preserving agent to ensure that the activity of colloidal silver will remain unvaried over time; in fact, continuous opening of the product could lead to contamination thereof with consequent reduction of the activity of silver.

Preserving agents, known in pharmaceutical techniques for the topical administration of the product, are used individually or in mixture, and in particular it is preferred to use potassium sorbate and/or imidazyl urea in an amount each of between approximately 0.1 g and 5 g per 1000 g of composition, more in particular each in an amount of approximately 1 g.

It is also possible to use natural aromas known in the art to render administration of the composition according to the present invention more agreeable. In particular, it is possible to use in the present invention aromas (strawberry, lemon and raspberry) in amounts each between approximately 0.1 g and 5 g per 1000 g of composition and in particular each in an amount of approximately 1 g.

The composition of the present invention can be prepared according to known techniques for nasal formulations.

Disclosed is the process for preparation of colloidal micronized silver used in the composition in question.

To obtain micronized silver to be dispersed in an aqueous solution, the process adopted was to strip or homogenize extremely pure metal silver at a high pressure. For this purpose apparatuses available on the market were used, such as a high-pressure press (for example, the APV Gaulin Homogenizer) and/or a high-pressure homogenizer (for example, the "Microfluidizer" manufactured by Microfludics), which afford the possibility of achieving the desired results through a number of micronization cycles.

The micronization was pushed to the point of achieving a silver that is so miniaturized that its particles in water remain in stable suspension: it has been found experimentally that said result is achieved when in the solution of water and silver the silver particles have a diameter of between 85 nm and 150 nm, with the majority of particles having a diameter of between 92 nm and 121 nm.

The tests on the stability of the product obtained were conducted at the laboratories of Qi Technologies s.r.l. of Pomezia (province of Roma) using the light-scattering technique.

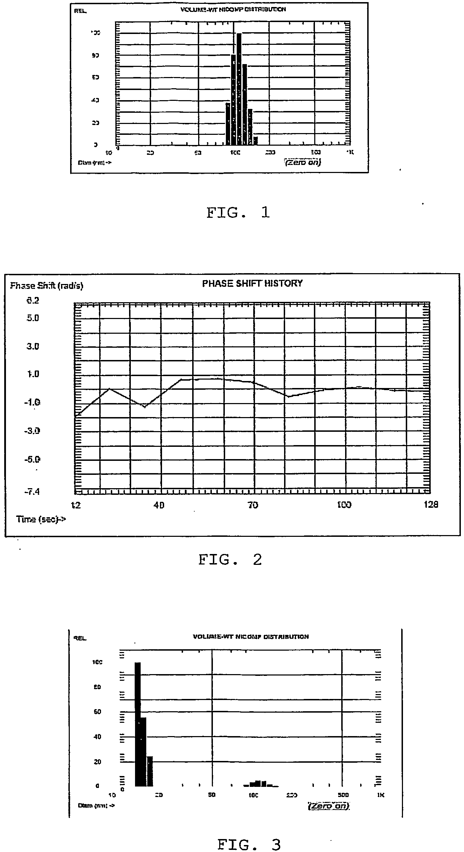

The graphs of Figures 1 to 6 illustrate the results of said tests.

As is known, the colloidal solutions are constituted by molecular aggregates dissolved in a solvent: said particles have diameters comprised between 1 nm and 200 nm and, when they are illuminated transversely, yield the Tyndall phenomenon.

For the analyses conducted, which were designed to determine the diameter of the silver particles dispersed in the aqueous medium, the method referred to as "Particle Sizing System" was used.

A series of specimens was prepared, constituted by colloidal solutions obtained by dispersing in water pure powdered metallic silver subjected to more or less intense micronization processes. The preparation of the colloidal solutions was carried out by dispersing the powdered micronized silver in water, in an amount of between approximately 10 ppm and 100 ppm and in particular in an amount of approximately 50 ppm.

On the colloidal solutions obtained the dimensions of the particles dispersed were measured, and measurement of the z-potential was made, in so far as the stability of a colloidal dispersion depends both upon the dimensions of the particles and upon the value and the sign of the electrical charge.

Appearing in the graphs illustrated in the attached figures are the results corresponding to a specimen prepared on February 25, 2008, filtered at time 0 with a 0.45-µm filter to eliminate the impurities and left under observation at room temperature and protected from direct light.

The results of said analysis can be inferred from the graph of Figure 1 where given on the abscissae is the diameter of the particles dispersed and on the ordinates the intensity of each component expressed in volume.

In the solution of water and silver the particles have a diameter of between 85 nm and 150 nm, whilst the majority of particles have a diameter of between 92 and 121 nm (mean diameter equal to 106.7 nm). Said distribution is considered very homogeneous and provides a first indication on the stability of the colloidal solution.

From an analysis of the graph of Figure 1 it is possible also to note a lack of sediment, this being an index of absence of interaction between the particles.

The particles of a colloidal solution are not at rest but are subject to Brownian motion (disorderly motion due to thermal agitation). When said particles collide they can coagulate to form larger particles, which, since they are heavier, precipitate: the absence of the sediment indicates that the silver particles have all the same electrical charge and hence, since they repel one another, do not coagulate nor do they settle to form a sediment.

Said information is also substantiated by the measurement of the z-potential performed on the same specimen.

From an analysis of the graph of Figure 2, it clearly emerges that the particles dispersed in the solution have a value of potential close to zero.

The report of said analysis indicates that the value of the z-potential is 0.09 mV. It may hence be said that there is no charge (this value is so small as to be considered zero): this prevents the particles from clustering, owing to the phenomenon of coagulation, and settling to form a single sediment.

Disclosed is the preparation of the pharmaceutical composition in question in the technical form of drops or spray for use in the disinfection of the nasal cavities.

The composition of the present invention can be prepared according to known techniques for nasal formulations.

Provided hereinafter is an example of formulation of the composition forming the subject of the invention, which is given purely for purposes of illustration, without this implying any limit to the scope of the invention.

### EXAMPLE OF FORMULATION FOR NASAL DROPS

| Components | Quantity |
|---|---|
| Microcolloidal silver | 50 ppm |
| Carboxymethyl beta glucan | 1 g |
| Potassium sorbate | 1 g |
| Imidazolydin urea | 1 g |
| Natural aroma | 1 g |
| Purified water | to reach 1000 g |

The end product presents as a slightly viscous, opalescent, brown liquid, with a pH of 6.90 ± 0.20.

In view of the information obtained on the specimen of just colloidal solution of water and micronized silver, it was decided to repeat said analysis on another two specimens of end product (where added to the colloidal solution of water and micronized silver were all the ingredients of the end formulation already tested from a microbiological standpoint):
Specimen A produced on June 23, 2008 filtered at time 0 with a 0.45-µm filter to eliminate the impurities and left under observation at room temperature and protected from direct light;
Specimen B produced on February 25, 2008 filtered at time 0 with a 0.45-µm filter to eliminate the impurities and left under observation at room temperature and protected from direct light.

The analyses conducted on the specimen produced on June 23, 2008 yielded the graph of Figure 3, where appearing on the abscissae is the diameter of the dispersed particles and appearing on the ordinates is the intensities of each component expressed in volume.

The first part of the graph highlights the presence of carboxymethyl beta glucan and indicates the presence of particles with diameter comprised between 13.5 and 15.7 nm (mean diameter 14.6 nm, with a standard deviation of 1.1 nm) and that said particles are present in terms of number of particles in a greater amount as compared to the silver particles.

The second part of the graph instead highlights the behaviour of the silver particles and indicates a maintenance of the characteristics found previously. The most important information that emerges from this graph is that, since there is no sediment, there does not exist any interaction between carboxymethyl beta glucan and silver; i.e., not even in this case is there any tendency towards coagulation and hence sedimentation. The product of specimen A is thus stable.

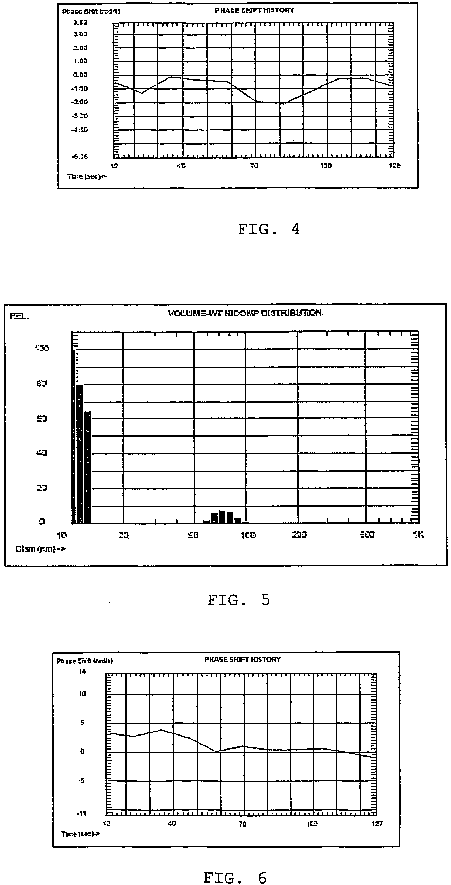

This statement is once again substantiated by an analysis of the z-potential of the same specimen. The graph of Figure 4 indicates in fact that also the end solution has a z-potential close to zero.

The value of the z-potential detected is 0.37 mV. Said value is small enough to consider the interaction between the particles as being absent; it shows, however, a slight increase with respect to the preceding analysis, an increase that can be put down to the presence of carboxymethyl beta glucan. The product is in any case stable.

The result of the analyses conducted on the specimen produced on February 25, 2008 indicates that the solution remains stable even after an ageing of approximately 4 months.

As may be seen in fact in graphs of Figures 5 and 6, respectively, there is no interaction in time between the particles (absence of a sediment) and the z-potential remains at a value close to zero (0.44 mV).

## Claims

1. A pharmaceutical composition for disinfection of the nasal mucosae, **characterized in that** it contains colloidal micronized silver with carboxymethyl beta glucan, and at least one preserving agent with possibly at least one natural aroma.

2. The composition according to Claim 1, **characterized in that** it contains colloidal micronized silver in an amount ranging between 10 and 100 ppm for a total of 1000 g of composition, preferably 50 ppm.

3. The composition according to Claim 1 or Claim 2, **characterized in that** it contains carboxymethyl beta glucan in an amount ranging between 0.1 to 5 g for a total of 1000 g of composition, preferably 1 g.

4. The pharmaceutical composition according to any one of Claims 1 to 3, in which the colloidal silver is micronized in the form of particles having a diameter of between 85 nm and 150 nm, with the majority of particles having a diameter of between 92 and 121 nm.

5. The pharmaceutical composition according to any one of Claims 1 to 4, in which potassium sorbate and/or the imidazyl urea are used as preserving agents each in an amount ranging between 0.1 and 5 g per 1000 g of composition, preferably 1 g.

6. The pharmaceutical composition according to any one of Claims 1 to 5, in which at least one natural aroma is used, each of said natural aroma being in an amount ranging between 0.1 to 5 g per 1000 g of composition, preferably 1 g.

7. The pharmaceutical composition according to Claims 1 to 6, **characterized in that** it is in the technical form of drops for disinfection of the nasal cavities.

8. The pharmaceutical composition according to Claims 1 to 6, **characterized in that** it is in the technical form of spray for disinfection of the nasal cavities.

9. The pharmaceutical composition according to anyone of the preceeding claims, in the form of nasal drops comprising:
| Components | Amount |
|---|---|
| Microcolloidal silver | 50 ppm |
| Carboxymethyl beta glucan | 1 g |
| Potassium sorbate | 1 g |
| Imidazolydin urea | 1 g |
| Natural aroma | 1 g |
| Purified water | to reach 1000 g |

10. Composition according to any one of Claims 1 to 9 for use in the disinfection of the nasal mucosae, in particular for children of up to twelve years of age.

## Patentansprüche

1. Pharmazeutische Zusammensetzung zur Desinfektion der Nasenschleimhäute, **dadurch gekennzeichnet, dass** sie kolloidales mikronisiertes Silber mit Carboxymethyl-beta-Glucan und zumindest ein Konservierungsmittel mit möglicherweise wenigstens einem natürlichen Aromastoff enthält

2. Zusammensetzung nach Anspruch 1, **dadurch gekennzeichnet, dass** sie kolloidales mikronisiertes Silber in einer Menge von zwischen 10 und 100 ppm, bezogen auf insgesamt 1000 g der Zusammensetzung, bevorzugt 50 ppm, enthält.

3. Zusammensetzung nach Anspruch 1 oder Anspruch 2, **dadurch gekennzeichnet, dass** sie Carboxymethyl-beta-Glucan in einer Menge von zwischen 0,1 - 5 g, bezogen auf insgesamt 1000 g der Zusammensetzung, bevorzugt 1 g, enthält.

4. Pharmazeutische Zusammensetzung nach einem der Ansprüche 1 - 3, in welcher das kolloidale Silber in Form von Teilchen mit einem Durchmesser von zwischen 85 nm und 150 nm mikronisiert vorliegt, wobei die Mehrheit der Teilchen einen Durchmesser von zwischen 92 und 121 nm aufweist.

5. Pharmazeutische Zusammensetzung nach einem der Ansprüche 1 - 4, in welcher Kaliumsorbat und/oder der Imidazylharnstoff als Konservierungsmittel eingesetzt werden, jeweils in einer Menge von zwischen 0,1 und 5 g pro 1000 g der Zusammensetzung, bevorzugt 1 g.

6. Pharmazeutische Zusammensetzung nach einem der Ansprüche 1 - 5, in welcher zumindest ein natürlicher Aromastoff eingesetzt wird, wobei jeder der natürlichen Aromastoffe in einer Menge von zwischen 0,1 - 5 g pro 1000 g der Zusammensetzung, bevorzugt 1 g, eingesetzt wird.

7. Pharmazeutische Zusammensetzung nach einem der Ansprüche 1 - 6, **dadurch gekennzeichnet, dass** sie in der technischen Form von Tropfen zur Desinfektion der Nasenhöhlen vorliegt.

8. Pharmazeutische Zusammensetzung nach einem der Ansprüche 1 - 6, **dadurch gekennzeichnet, dass** sie in der technischen Form eines Sprays zur Desinfektion der Nasenhöhlen vorliegt.

9. Pharmazeutische Zusammensetzung nach einem der vorhergehenden Ansprüche in Form nasaler Tropfen, umfassend:
| Bestandteile | Menge |
|---|---|
| Mikrokolloidales Silber | 50 ppm |
| Carboxymethyl-beta-Glucan | 1 g |
| Kaliumsorbat | 1 g |
| Imidazolydinharnstoff | 1 g |
| Natürlicher Aromastoff | 1 g |
| Gereinigtes Wasser | auf 1000 g |

10. Zusammensetzung nach einem der Ansprüche 1 - 9 zur Verwendung zur Desinfektion der Nasenschleimhäute, insbesondere für Kinder bis zu einem Alter von 12 Jahren.

## Revendications

1. Composition pharmaceutique destinée à la désinfection des muqueuses nasales, **caractérisée en ce qu'**elle contient de l'argent micronisé colloïdal avec du bêta-glucane de carboxyméthyle, et au moins un agent conservateur avec éventuellement au moins un arôme naturel.

2. Composition selon la revendication 1, **caractérisée en ce qu'**elle contient de l'argent micronisé colloïdal dans une quantité comprise entre 10 et 100 ppm pour un total de 1000 g de composition, de préférence 50 ppm.

3. Composition selon la revendication 1 ou la revendication 2, **caractérisée en ce qu'**elle contient du bêta-glucane de carboxyméthyle dans une quantité comprise dans la plage de 0,1 à 5 g pour un total de 1000 g de composition, de préférence 1 g.

4. Composition pharmaceutique selon l'une quelconque des revendications 1 à 3, dans laquelle l'argent colloïdal est micronisé sous la forme de particules ayant un diamètre compris entre 85 nm et 150 nm, avec la majorité de particules ayant un diamètre compris entre 92 nm et 121 nm.

5. Composition pharmaceutique selon l'une quelconque des revendications 1 à 4, dans laquelle du sorbate de potassium et/ou l'imidazyl urée sont utilisés comme agents conservateurs chacun dans une quantité comprise entre 0,1 et 5 g pour un total de 1000 g de composition, de préférence 1 g.

6. Composition pharmaceutique selon l'une quelconque des revendications 1 à 5, dans laquelle au moins un arôme naturel est utilisé, chacun desdits arômes naturels étant dans une quantité comprise dans la plage de 0,1 à 5 g pour un total de 1000 g de composition, de préférence 1 g.

7. Composition pharmaceutique selon les revendications 1 à 6, **caractérisée en ce qu'**elle est sous la forme technique de gouttes pour la désinfection des cavités nasales.

8. Composition pharmaceutique selon les revendications 1 à 6, **caractérisée en ce qu'**elle est sous la forme technique de pulvérisation pour la désinfection des cavités nasales.

9. Composition pharmaceutique selon l'une quelconque des revendications précédentes, sous la forme de gouttes nasales comprenant :
| Composants | Quantité |
|---|---|
| Argent microcolloïdal | 50 ppm |
| Bêta-glucane de carboxyméthyle | 1 g |
| Sorbate de potassium | 1 g |
| Imidazolidinyl urée | 1 g |
| Arôme naturel | 1 g |
| Eau purifiée | pour atteindre 1000 g |

10. Composition selon l'une quelconque des revendications 1 à 9 pour l'usage dans la désinfection des muqueuses nasales, en particulier pour des enfants jusqu'à un âge de douze ans.
